## Europäisches Patentamt

## European Patent Office

## Office européen des brevets

(19)

(11) Veröffentlichungsnummer: **0 119 321
B1**

(12) **EUROPÄISCHE PATENTSCHRIFT**

(45) Veröffentlichungstag der Patentschrift:
**07.10.87**

(51) Int. Cl.⁴: **A 61 F 2/34**

(21) Anmeldenummer: **83113024.0**

(22) Anmeldetag: **23.12.83**

(54) **Hüftgelenkspfanne.**

(30) Priorität: **09.03.83 CH 1260/83**

(43) Veröffentlichungstag der Anmeldung:
**26.09.84 Patentblatt 84/39**

(45) Bekanntmachung des Hinweises auf die Patenterteilung:
**07.10.87 Patentblatt 87/41**

(84) Benannte Vertragsstaaten:
**AT DE FR GB IT NL SE**

(56) Entgegenhaltungen:
**DE - B - 2 205 400
FR - A - 2 242 065
FR - A - 2 315 903
FR - A - 2 429 009
GB - A - 1 215 737**

(73) Patentinhaber: **GEBRÜDER SULZER
AKTIENGESELLSCHAFT, Zürcherstrasse 9,
CH-8401 Winterthur (CH)**
Patentinhaber: **Protek AG, Stadtbachstrasse 64,
CH-3012 Bern (CH)**

(72) Erfinder: **Niederer, Peter Gino, P.O. Box 31178, Santa
Barbara California 93130 (US)**
Erfinder: **Frey, Otto, Wallrütistrasse 56,
CH-8400 Winterthur (CH)**

(74) Vertreter: **Dipl.-Ing. H. Marsch Dipl.-Ing. K. Sparing
Dipl.-Phys.Dr. W.H. Röhl Patentanwälte,
Retheistrasse 123, D-4000 Düsseldorf (DE)**

## Beschreibung

Die Erfindung betrifft eine Hüftgelenkspfanne mit einem die Pfannenschale enthaltenden Pfannenkörper, dessen Aussenmantel in Umfangsrichtung eine ringförmige Oberflächenstruktur trägt und von einem geschlossenen Ring aus Metall oder einer Metallegierung umgeben ist, der den Pfannenkörper unter Vorspannung hält.

Eine Hüftgelenkspfanne der vorstehend genannten Art ist aus der FR–A–2 429 009 bekannt. Diese Konstruktion besteht aus einem keramischen Pfannenkörper, der durch einen Ring aus Metall unter einer radialen Vorspannung gehalten ist. Der Ring hat die Aufgabe, den zylindrischen Aussenmantel des Pfannenkörpers aus Keramik mit einer Struktur – beispielsweise einer Oberflächenrauhigkeit oder einem Gewinde – zu versehen, durch die die Fixierung der Prothese im Becken verbessert wird; die radiale Vorspannung dient dazu, eine innige Verbindung zwischen Keramikinnenkörper und dem Ring zu gewährleisten.

Weiterhin ist eine Vielzahl künstlicher Hüftgelenkspfannen mit halbkugel- oder kegelstrumpfförmigem Pfannenkörper bekannt (siehe z.B. CH–A–593 054 oder CH–A–568 753). Wegen der Gleiteigenschaften werden derartige Hüftgelenkspfannen sehr häufig aus Kunststoff, insbesondere aus Polyäthylen, hergestellt.

In der Endoprothetik ist man bestrebt, die einen Fremdkörper im menschlichen Knochen und Gewebe bildenden künstlichen Hüftgelenkspfannen möglichst klein im Volumen zu halten. Dieses Bestreben führt zu relativ dünnwandigen Pfannenkörpern, die relativ nachgiebig sind und die Beckenbewegungen auf die zueinander kongruenten Gleitflächen übertragen; diese Bewegungen führen zu dem sogenannten «Kirschkern»-Effekt, worunter man das «Herausarbeiten» eines festen Kerns aus einem weicheren Material bei Relativbewegungen zueinander versteht. Sofern die Pfannenkörper aus Kunststoff bestehen, neigen sie unter den dauernden Belastungen weiterhin zu plastischen Verformungen, die zu Veränderungen ihrer Schalenfläche führen. Dadurch wird die Kongruenz der eine Gleitfläche des Gelenkes bildenden Schalenoberfläche mit der anderen Gleitfläche, die von der Oberfläche des Gelenkkopfes gebildet wird, gestört, was zu erhöhtem Abrieb führt.

Es besteht daher die Forderung, dass die Hüftgelenkspfanne als Ganzes möglichst fest und starr sein soll, so dass sie sich bei Bewegungen des Gelenkes nicht verformt. Diese Forderung hat zu einer Anzahl von Konstruktionen geführt, bei denen eine innere Kunststoffschale von einer im allgemeinen metallischen Aussenschale umhüllt und in dieser gehalten wird – siehe z.B. GB–A–1 215 737 –. Damit wird zwar der Pfannenkörper aus Kunststoff gegen den direkten Einfluss der Beckenbewegungen geschützt; bei diesen Konstruktionen treten aber Relativbewegungen zwischen dem inneren Pfannenkörper und seiner Aussenschale auf, die zu Verschleiss und Abrieb führen, der zwischen die beiden Gleitflächen gelangen kann.

Aufgabe der Erfindung ist es, eine starre Hüftgelenkspfanne zu schaffen, die gegen die Beckenbewegungen widerstandsfähig ist und die Gleiteigenschaften von Kunststoffpfannen besitzt; weiterhin muss die Verbindung zwischen Kunststoff-Pfannenkörper und dem Ring so gestaltet sein, dass Relativbewegungen zwischen beiden und ein Fliessen des Kunststoffes verhindert werden.

Diese Aufgabe wird erfindungsgemäss dadurch gelöst, dass der halbkugel- oder kegelstumpfförmige Pfannenkörper aus Kunststoff besteht und seine ringförmige Oberflächenstruktur einen stufenförmigen Absatz aufweist, auf dem der geschlossene Verstärkungsring gelagert ist, und dass ferner die in Richtung der Rotations-Symmetrieachse des Pfannenkörpers verlaufende Flanke des stufenförmigen Absatzes und die Gegenflanke des Verstärkungsringes mit dieser Achse einen Winkel bilden und sich in Richtung zum Pol des Pfannenkörpers konisch erweitern.

Der aus Kunststoff gefertigte Pfannenkörper wird auf diese Weise so versteift, dass sich eine starre und feste Pfanne ergibt. Durch die Vorspannung, mit der der Verstärkungsring, der aus einem der in der Implantat-Technik üblichen Metalle oder einer Metall-Legierung besteht, auf dem Kunststoffkörper sitzt, wird eine Relativbewegung zwischen beiden Komponenten verhindert. Die konische Form der beiden Flanken bewirkt, dass die Vorspannung nicht nur rein radial wirkt, sondern auch eine axiale Komponente aufweist; diese erhöht den Widerstand des Pfannenkörpers gegen ein Fliessen des Kunststoffes und gegen Lockern oder «Abrutschen» des Ringes in Richtung der Verjüngung des Pfannenkörpers, d.h. polwärts, und presst den Ring gegen den Anschlag oder Absatz des Pfannenkörpers.

Es kann eine Mehrzahl von Verstärkungsringen mit zugeordneten stufenförmigen Absätzen vorgesehen sein.

Die Verankerung der Pfanne im Beckenknochen mit einem Zementbett oder zementfrei lässt sich effektiver gestalten, wenn die Verstärkungsringe aus dem Umriss des Pfannenkörpers herausragen oder sogar nach aussen vorspringende Ansätze haben.

Im folgenden wird die Erfindung anhand von Ausführungsbeispielen näher erläutert.

Fig. 1 zeigt in der linken Hälfte im Schnitt und in der rechten Hälfte in der Ansicht eine erste Ausführungsform der neuen Hüftgelenkspfanne;

Fig. 2 ist in gleicher Darstellung ein zweites Beispiel, während

Fig. 3–5 in grösserem Massstab und im Schnitt in je einem Ausschnitt Beispiele mit unterschiedlich geformten Verstärkungsringen darstellen, wobei Fig. 3 einen Ausschnitt aus Fig. 2 wiedergibt.

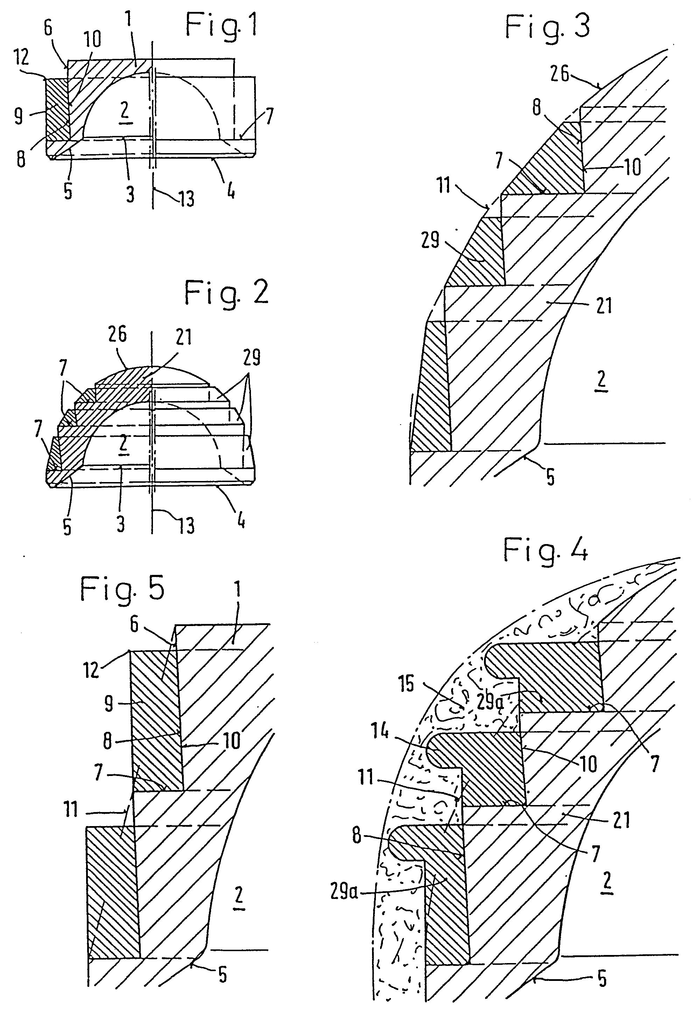

Die Hüftgelenkspfanne nach Fig. 1 hat einen Pfannenkörper 1, in dessen Basis die eigentliche Pfannenschale 2 in Form einer hohlen Halbkugel eingearbeitet ist, die den Gelenkkopf einer nicht dargestellten Femurkopfprothese aufnimmt. Da der Basisdurchmesser 3 der Halbkugel 2 nicht mit der Basisfläche 4 des Pfannenkörpers 1 zusammenfällt, sondern dieser gegenüber in den Pfannenkörper 1 hineinverschoben ist, geht die hohle Halbkugel von ihrem Basisdurchmesser 3 aus nach aussen in einen sich erweiternden Konus 5 über.

Erfindungsgemäss ist in dem Aussenmantel 6 des Pfannenkörpers 1 ein Absatz 7 eingearbeitet, dessen Seitenflanke 8 entgegen der Verjüngung des Pfannenkörpers 1 konisch verläuft. Auf diesem Absatz 7 ist ein Verstärkungsring 9 «aufgeschrumpft», der dem Pfannenkörper 1, ähnlich wie ein Fassreifen, eine hohe Festigkeit und Steifigkeit verleiht. Seine zur Seitenflanke 8 des Pfannenkörpers 1 gerichtete Gegenflanke 10 ist ebenfalls konisch geformt und in ihrem Konus an denjenigen der Seitenflanke 8 angepasst.

Das Aufschrumpfen des Verstärkungsringes 9 kann beispielsweise durch einfaches Aufpressen oder auch mit Hilfe einer herkömmlichen Technik erfolgen, bei der der Pfannenkörper 1 beispielsweise vor dem Aufsetzen des Verstärkungsringes 9 tiefgekühlt wird, dessen Durchmesser etwas kleiner, beispielsweise um 0,5 mm, als der Durchmesser des Pfannenkörpers 1 im Bereich des Absatzes 7 bei «Betriebstemperatur» ist, so dass der Pfannenkörper 1 «während des Betriebs» unter Vorspannung gehalten ist.

Wie besonders Fig. 5 verdeutlicht, die einen Ausschnitt aus einer ähnlichen Pfanne zeigt, bei der lediglich statt einem Absatz 7 zwei mit Verstärkungsringen 9 besetzte Absätze 7 vorhanden sind, ragen die Verstärkungsringe 9 in diesem Ausführungsbeispiel mit ihren äusseren Bereichen aus dem kegelstumpfförmigen Pfannenkörper 1 heraus, dessen fiktive seitliche Begrenzungslinie mit 11 bezeichnet ist. Mit Hilfe dieser herausragenden Teile wird die Fläche für eine Verankerung der Gesamtpfanne und damit ihre Fixierung verbessert. Selbstverständlich sind dabei die Kanten 12 der Ringe 9 nicht scharfkantig ausgebildet, um Spitzenbelastungen, die zu Rissen im Beckenknochen und/oder im Zementbett führen können, zu vermeiden.

Die Ausführungsform nach Fig. 2 und 3 unterscheidet sich von derjenigen nach Fig. 1 nur in einigen Details. So ist der Aussenmantel 26 ihres Pfannenkörpers 21 halbkugelförmig. Statt eines Absatzes 7 sind deren drei vorgesehen, die unter sich unterschiedliche Höhen in Richtung der Pfannenachse 13 haben. Schliesslich sind die Begrenzungen der Verstärkungsringe 29 nach aussen an die Umrisslinie 11 des Pfannenkörpers 21 angepasst, wie besonders aus Fig. 3 zu erkennen ist.

Bei der Variante nach Fig. 4, die im wesentlichen derjenigen nach Fig. 2 und 3 entspricht, haben die Verstärkungsringe 29a nach aussen gerichtete Vorsprünge 14, mit denen sie in ein Zementbett 15 hineinragen, um die Festigkeit der Verankerung in diesem Zementbett 15 zu erhöhen.

Selbstverständlich ist noch eine Vielzahl weiterer Varianten für die Querschnittsform der Verstärkungsringe 9 möglich, um den Halt der Pfanne im Knochen oder in einem Zementbett zu erhöhen.

## Patentansprüche

1. Hüftgelenkspfanne mit einem die Pfannenschale (2) enthaltenden Pfannenkörper (1, 21), dessen Aussenmantel (6, 26) in Umfangsrichtung eine ringförmige Oberflächenstruktur trägt und von einem geschlossenen Ring (9, 29, 29a) aus Metall oder einer Metallegierung umgeben ist, der den Pfannenkörper (1, 21) unter Vorspannung hält, dadurch gekennzeichnet, dass der halbkugel- oder kegelstumpfförmige Pfannenkörper (1, 21) aus Kunststoff besteht und seine ringförmige Oberflächenstruktur einen stufenförmigen Absatz (7) aufweist, auf dem der geschlossene Verstärkungsring (9, 29, 29a) gelagert ist, und dass ferner die in Richtung der Rotations-Symmetrieachse (13) des Pfannenkörpers (1, 21) verlaufende Flanke (8) des stufenförmigen Absatzes (7) und die Gegenflanke (10) des Verstärkungsringes (9, 29, 29a) mit dieser Achse (13) einen Winkel bilden und sich in Richtung zum Pol des Pfannenkörpers (1, 21) konisch erweitern.

2. Hüftgelenkspfanne nach Anspruch 1, dadurch gekennzeichnet, dass eine Mehrzahl von Verstärkungsringen (9, 29, 29a) mit zugeordneten stufenförmigen Absätzen (7) vorgesehen ist.

3. Hüftgelenkspfanne nach Anspruch 2, dadurch gekennzeichnet, dass die Verstärkungsringe (9, 29a) aus dem Umriss (11) des Pfannenkörpers (1, 21) herausragen.

4. Hüftgelenkspfanne nach Anspruch 3, dadurch gekennzeichnet, dass die Verstärkungsringe (29a) nach aussen vorspringende Ansätze (14) haben.

## Claims

1. An acetabulum having a socket body (1, 21) which contains the acetabulum shell (2) and whose outer generated surface (6, 26) carries an annular surface structure in the peripheral direction and has around it a closed ring (9, 29, 29a) made of metal or of a metal alloy, the ring putting the socket body (1, 21) under pre-stress, characterised in that the hemispherical or frustumshaped socket body (1, 21) is made of plastics and its annular surface structure has a stepped shoulder (7) on which the closed reinforcing ring (9, 29, 29a) is mounted; and that flank (8) of the stepped shoulder (7) which extends in the direction of the axis (13) of rotational symmetry of the socket body (1, 21), and the matching flank (10) of the reinforcing ring (9, 29, 29a) form an angle with the latter axis (13) and widen conically towards the pole of the socket body (1, 21).

2. An acetabulum according to claim 1, characterised in that a number of reinforcing rings (9, 29, 29a) with associated stepped shoulders (7) are provided.

3. An acetalbulum according to claim 2, characterised in that the reinforcing rings (9, 29a) project beyond the contour (11) of the socket body (1, 21).

4. An acetabulum according to claim 3, characterised in that the reinforcing rings (29a) have outwardly extending projections (14).

## Revendications

1. Cuvette coxofémorale, présentant un corps (1, 21) qui renferme la calotte (2) de cette cuvette et dont l'enveloppe externe (6, 26) porte, dans le sens périphérique, une structure superficielle annulaire et est entourée par un anneau fermé (9, 29, 29a) en métal ou en un alliage métallique, maintenant le corps (1, 21) de la cuvette sous une tension préalable, caractérisée par le fait que le corps hémisphérique ou tronconique (1, 21) de la cuvette consiste en une matière plastique, sa structure superficielle annulaire présentant un décrochement (7) en forme de gradin sur lequel l'anneau fermé de renforcement (9, 29, 29a) est placé; et par le fait que le flanc (8) du décrochement (7) en forme de gradin, s'étendant en direction de l'axe (13) de symétrie de rotation du corps (1, 21) de la cuvette, et le flanc complémentaire (10) de l'anneau de renforcement (9, 29, 29a) forment en outre un angle avec ledit axe (13), et s'évasent tronconiquement en direction du pôle du corps (1, 21) de la cuvette.

2. Cuvette coxofémorale selon la revendication 1, caractérisée par le fait que sont prévus de multiples anneaux de renforcement (9, 29, 29a), auxquels des décrochements (7) en forme de gradins sont associés.

3. Cuvette coxofémorale selon la revendication 2, caractérisée par le fait que les anneaux de renforcement (9, 29a) dépassent au-delà du contour (11) du corps (1, 21) de la cuvette.

4. Cuvette coxofémorale selon la revendication 3, caractérisée par le fait que les anneaux de renforcement (29a) possèdent des appendices (14) en saillie vers l'extérieur.

Fig. 1

Fig. 2

Fig. 3

Fig. 4

Fig. 5